# EUROPEAN PATENT APPLICATION

(11) **EP 3 901 269 A1**
(43) Date of publication of application: **27.10.2021**
(21) Application number: 21170517.3
(22) Date of filing: 26.04.2021
(51) Int. Cl.: C12P 7/24, C12P 17/02, C12R 1/85

(54) **METHOD FOR PREPARING NATURAL DELTA-DECALACTONE AND DELTA-DODECALACTONE BY BIOREDUCTION OF MASSOIA OIL**

(30) Priority: 24.04.2020 CN 202010331414
(71) Applicant: Xiamen Oamic Biotechnology Co., Ltd., Xiamen, Fujian 361026 (CN)
(72) Inventor: ZENG, Shichao, Xiamen, 361026 (CN); ZHAO, Xiaolan, Xiamen, 361026 (CN); XIAO, Chunyan, Xiamen, 361026 (CN); LIN, Xiaohui, Xiamen, 361026 (CN); XING, Chenguang, Xiamen, 361026 (CN); LIU, Gang, Xiamen, 361026 (CN); ZHAO, Xijing, Xiamen, 361026 (CN)
(74) Representative: Verscht, Thomas Kurt Albert

(57) **Abstract**

The present disclosure discloses a method for preparing natural delta-decalactone and delta-dodecalactone by bioreduction of Massoia Oil, which comprises (1) Strain activation, (2) Preparation of seed culture solution, (3) Fermentation and conversion, (4) Extraction, (5) Solvent recycle, and (6) Rectification. The present disclosure has a higher space-time yield relative to the existing techniques and obtains a higher product concentration. The Saccharomyces pastorianus OMK-70 was obtained by multiple rounds of strain mutagenesis of the present disclosure is used to ferment and reduce δ-lactone of 5-hydroxy-2-decenoic acid and δ-lactone of 5-hydroxy-2-dodecenoic acid in Massoia Oil. Which has a higher effectiveness, thus the production cost is greatly reduced. It has good industrial application prospects.

## Description

### FIELD OF THE DISCLOSURE

The present disclosure relates to fermentation engineering, and in particular, relates to a method for preparing natural delta-decalactone and delta-dodecalactone by bioreduction of Massoia Oil.

### BACKGROUND OF THE DISCLOSURE

Delta-decalactone and delta-dodecalactone have a coconut aroma and have a creamy aroma at low concentrations. They are found in fruits, such as coconuts and raspberries. They are important raw materials for formulating milk, cream, coconut, strawberry, and peach flavors. The market demand is large.

At present, the delta-decalactone and the delta-dodecalactone on the market are mainly chemically synthesized products. With progress and development of the society, a willingness of human beings to return to nature has become stronger and stronger, which promotes the application of natural flavors in the food industry. Compared with the chemically synthesized products, natural flavors derived from biotransformation of natural substances have higher market acceptance and added value.

In the existing techniques, production of natural delta-decalactone and delta-dodecalactone mainly relies on microbial fermentation to reduce δ-lactone of 5-hydroxy-2-decenoic acid and δ-lactone of 5-hydroxy-2-dodecenoic acid respectively. Due to low space-time yield and low product concentration, which do not have industrial application prospect. For example, in the technical solution disclosed in US6187741, fermentation of *Saccharomyces cerevisiae* to reduce δ-lactone of 5-hydroxy-2-decenoic acid for 65 hours only produces 7.37 g/L delta-decalactone, and fermentation to reduce δ-lactone of 5-hydroxy-2-dodecenoic acid only produces 4 g/L delta-dodecalactone in 60 hours. The technical solution disclosed in US6025170 using *Clostridium tyrobutyricum* 1-776 resting cells only produces 7 g/L delta-decalactone in 40 hours and only 9.6 g/L delta-dodecalactone in 44 hours. In the technical solution disclosed in US5763233, using *Pseudomonas putida* ATCC 33015 to reduce δ-lactone of 5-hydroxy-2-decenoic acid for 48 hours can only produce 10.9 g/L delta-decalactone.

### BRIEF SUMMARY OF THE DISCLOSURE

An object of the present disclosure is to provide a method for preparing natural delta-decalactone and delta-dodecalactone by bioreduction of Massoia Oil.

Another object of the present disclosure provides *Saccharomyces pastorianus* OMK-70 used in the above method.

A technical solution of the present disclosure is as follows.

A method for preparing natural delta-decalactone and delta-dodecalactone by bioreduction of Massoia Oil comprises the following steps.

(1) culturing *Saccharomyces pastorianus* OMK-70 on a solid slant culture medium and a seed culture medium in sequence to obtain a seed culture solution, the *Saccharomyces pastorianus* OMK-70 was deposited in China Center for Type Culture Collection (Wuhan University, Wuhan, China) on December 27, 2019, with a deposit number of CCTCC NO: M 20191123;

(2) inoculating the seed culture solution into a fermentation medium and cultivating, adding Massoia oil in batches after strains grow to an early logarithmic stage, and continuously adding glucose and continually fermenting;

(3) adding butyl acetate into a culture solution obtained in step (2), completely stirring and reacting, leaving to stand to generate layers, and then acquiring an upper oil phase; and

(4) recycling the butyl acetate in the upper oil phase, rectifying a pot bottom by controlling a rectification temperature, and collecting fractions at different temperatures to obtain the natural delta-decalactone and the delta-dodecalactone.

In a preferred embodiment, which comprises the following steps:
(1) strain activation: streaking the *Saccharomyces pastorianus* OMK-70 from a glycerol tube preserved at low temperature on the solid slant culture medium and static culturing at 28 °C-30 °C for 48-72 hours to obtain the activated strains; and
(2) preparation of the seed culture solution: inoculating the activated strains obtained in step (1) into the seed culture medium and shake culturing at 28 °C-30 °C and 200-250 rpm to grow to the early logarithmic stage to obtain the seed culture solution;
(3) fermentation and conversion: inoculating the seed culture solution obtained in step (2) into the fermentation medium at a volume ratio of 5-10% and fermenting for 6-12 hours in a condition that pH is 4.8-6.4, 28 °C-30 °C, a stirring speed is 100-600 rpm, and an aeration volume is 1:0.5, adding the Massoia oil in batches after the strains grow to the early logarithmic stage, and continuously adding glucose and continually fermenting for 48-72 hours;
(4) extraction: adding the butyl acetate to a culture broth obtained in step (3) at a volume ratio of 0.8-1.2:0.8-1.2, stirring at 30 °C-40 °C and 200-300 rpm for 1-2 hours, leaving to stand to generate the layers, and then acquiring the upper oil phase;
(5) solvent recycling: transferring the upper oil phase obtained in step (4) to a distillation flask to recycle the butyl acetate at an atmosphere pressure; and
(6) rectification: transferring a pot bottom obtained in step (5) into a rectification tower and rectifying by controlling the rectification temperature, and collecting the fractions at the different temperatures to obtain the natural delta-decalactone and the delta-dodecalactone.

In a preferred embodiment, a composition of the solid slant culture medium in mass percentage comprises: peptone 0.5-5%, yeast extract 0.1-3%, glucose 1-5%, agar 0.5-5%, and a solvent is water.

In a preferred embodiment, the composition of the solid slant culture medium in mass percentage comprises: peptone 2.5%, yeast extract 1.5%, glucose 2.5%, agar 1.5%, and a balance is the water.

In a preferred embodiment, a composition of the seed culture medium in mass percentage comprises: peptone 0.5-5%, yeast extract 0.1-3%, and glucose1-5%, and a solvent is water.

In a preferred embodiment, the composition of the seed culture medium in mass percentage comprises: peptone 2.5%, yeast extract 1.5%, glucose 2.5%, and a balance is the water.

In a preferred embodiment, a composition of the fermentation medium in mass percentage comprises: glucose 2.0-5.0%, ammonium dihydrogen phosphate 0.5-1.0%, potassium dihydrogen phosphate 0.1-0.5%, magnesium sulfate 0.05-0.2%, calcium sulfate 0.05-0.1%, yeast extract powder 0.1-1.0%, and a solvent is water.

In a preferred embodiment, the composition of the fermentation medium in mass percentage comprises: glucose 2%, ammonium dihydrogen phosphate 1%, potassium dihydrogen phosphate 0.5%, magnesium sulfate 0.2%, calcium sulfate 0.1%, yeast extract powder 0.5%, and a balance is the water.

Another technical solution of the present disclosure is as follows.

*Saccharomyces pastorianus* OMK-70 deposited in China Center for Type Culture Collection (Wuhan university, Wuhan, China) on December 27, 2019, with a deposit number of CCTCC NO: M 20191123.

A method for preparing natural delta-decalactone and natural delta-dodecalactone by bioreduction of Massoia Oil using the *Saccharomyces pastorianus* OMK-70.

Compared with the existing techniques, the technical solution has the following advantages.

The *Saccharomyces pastorianus* OMK-70 of the present disclosure is used to ferment and reduce δ-lactone of 5-hydroxy-2-decenoic acid and δ-lactone of 5-hydroxy-2-dodecenoic acid in Massoia Oil, with a space-time yield of 1.1g/(L^{∗}h) and a product concentration of 52.1 g/L for delta-decalactone, which are much higher than the existing production process, thus the production cost is greatly reduced. It has good industrial application prospects.

### BRIEF DESCRIPTION OF THE DRAWINGS

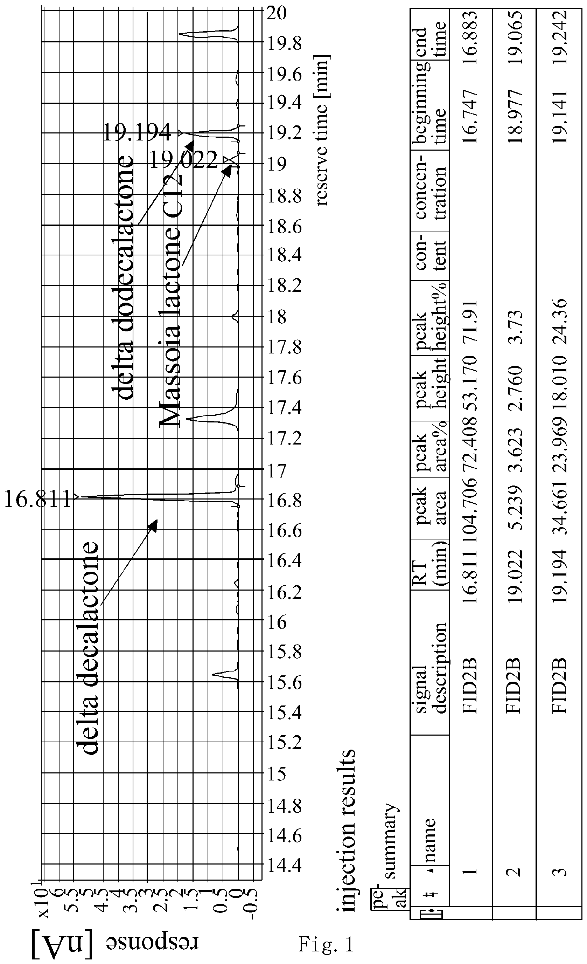

FIG. 1 is a GC graph of Massoia Oil fermented and reduced by *Saccharomyces pastorianus* OMK-70 in Embodiment 2 of the present disclosure.

### DETAILED DESCRIPTION OF THE EMBODIMENTS

The technical solution of the present disclosure will be further described below in combination with the accompanying drawings and embodiments.

### Embodiment 1: Production of natural delta-decalactone and delta-dodecalactone (fermentation in a triangular shaking flask)

(1) Strain acquisition
   A strain used to catalyze a reduction of δ-lactone of 5-hydroxy-2-decenoic acid to generate delta-decalactone was isolated from a marine mud bed, which was identified as *Saccharomyces pastorianus* by 18S rDNA and ITS sequencing and was named *Saccharomyces pastorianus* OMK-70. The strain was deposited in China Center for Type Culture Collection (Wuhan University, Wuhan, China), on December 27, 2019, with a deposit number of CCTCC NO: M20191123.
(2) Strain activation: *Saccharomyces pastorianus* OMK-70 was taken from a -80 °C glycerol tube, streaked on a solid slant culture medium, and cultured at 30 °C for 60 hours to then obtain activated strains.
   A composition of the solid slant culture medium in mass percentage was as follows: peptone 2.5%, yeast extract 1.5%, glucose 2.5%, agar 1.5%, and a balance was water.
(3) Preparation of seed culture solution: the activated strains were inoculated into a 500 mL triangular shaking flask containing 30 mL of a seed culture medium and was cultivated at 29 °C and 250 rpm for 4-6 hours to obtain the seed culture solution.
   A composition of the seed culture medium in mass percentage was as follows: peptone 2.5%, yeast extract 1.5%, and glucose 2.5%. The solvent was water, and the initial pH was 7.5. It was sterilized at 115 °C for 30 minutes.
(4) Fermentation and conversion: 5 mL of the seed culture solution was inoculated in a 500 mL triangular shaking flask containing 50 mL of a fermentation medium and was fermented at 29 °C and 250 rpm for 8 hours. After the strains entered a logarithmic growth phase, 20 g/L Massoia Oil (which includes 65% δ-lactone of 5-hydroxy-2-decenoic acid and 17% δ-lactone of 5-hydroxy-2-dodecenoic acid) was added, and it was continuously fermented at 29 °C and 250 rpm for 72 hours during which 10 g/L glucose was added every 12 hours.
(5) Extraction: after the fermentation was completed, which was heated to 60 °C for inactivation, and the natural delta-decalactone and the delta-dodecalactone in the fermentation broth were extracted by butyl acetate having an equal volume. It was stirred at 30 °C-40 °C and 200-300 rpm for 1-2 hours and was left to stand to generate layers. The upper oil phase was then acquired, and contents were analyzed by GC. A concentration of the delta-decalactone was 12.6 g/L with a conversion rate of 96.9%, and a concentration of the delta-dodecalactone was 2.9 g/L with a conversion rate of 85.3%.

A composition of the fermentation medium in mass percentage was as follows: glucose 2%, ammonium dihydrogen phosphate 1%, potassium dihydrogen phosphate 0.5%, magnesium sulfate 0.2%, calcium sulfate 0.1%, and yeast extract powder 0.5%. The solvent was water, and the initial pH was 7.5. It was sterilized at 115 °C for 30 minutes.

### Embodiment 2: Production of natural delta-decalactone and delta-dodecalactone (fermentation in a fermenter)

Steps (1) and (2) are the same as Embodiment 1.
(3) Preparation of seed culture solution: the activated strains were inoculated in a 500 mL triangular shaking flask containing 50 mL of a seed culture medium and were incubated at 30 °C and 250 rpm for 10-12 hours to obtain a first-stage seed solution. All of the 50 mL first-stage seed solution was inoculated in a 10 L seed tank containing 6 L of the seed culture medium and was cultivated for 6-8 hours at 30 °C at a condition in which a stirring speed was 250 rpm and an aeration volume was 1:0.5 to obtain a second-stage seed liquid.
   A composition of the seed culture medium was the same as Embodiment 1.
(4) Fermentation and conversion: 16.2 L of the fermentation medium was added in a 30L fermenter and was sterilized at 115 °C for 30 minutes. The second-stage seed liquid was transferred into a 30 L fermenter based on a 10% inoculum dose, cultivated in a cultivation condition in which a temperature was 30 °C, a stirring speed was 400 rpm, and an aeration ratio was 1:0.5. After being fermented for 8 hours, 20 g/L Massoia oil was added and was continually fermented, 70% glucose solution was added at a flow rate of 20 g/h, and then 20 g/L Massoia oil was respectively added at 16 hours, 26 hours, and 40 hours of the fermentation. A total period lasts for 55 hours.
(5) Extraction: after the fermentation was completed, which was heated to 60 °C for inactivation, and the natural delta-decalactone and the delta-dodecalactone in the fermentation broth were extracted by butyl acetate having an equal volume. It was stirred at 30 °C-40 °C and 200-300 rpm for 1-2 hours and was left to stand to generate layers. An upper oil phase was then acquired, and contents were analyzed by GC. A concentration of the delta-decalactone was 52.1 g/L with a conversion rate of 100.0%, and a concentration of the delta-dodecalactone was 11.8 g/L with a conversion rate of 86.9%. (A GC graph is shown in Fig. 1).
(6) Solvent recycle and rectification: the upper oil phase was transferred into a distillation flask to recycle the butyl acetate at atmospheric pressure. After that, a pot bottom was transferred into a rectification tower. A rectification temperature was controlled, and fractions at different temperatures were collected to obtain 821.3g of the natural delta-decalactone with a yield of 87.7%, a purity of 97%, and an *ee* value of 92.1% (R) and 186.5 g the natural delta-dodecalactone with a yield of 76.2%, a purity of 98%, and an *ee* value of 91.8% (R).

The invention may be summarized as follows: The present disclosure discloses a method for preparing natural delta-decalactone and delta-dodecalactone by bioreduction of Massoia Oil, which comprises (1) Strain activation, (2) Preparation of seed culture solution, (3) Fermentation and conversion, (4) Extraction, (5) Solvent recycle, and (6) Rectification. The present disclosure has a higher space-time yield relative to the existing techniques and obtains a higher product concentration. The Saccharomyces pastorianus OMK-70 was obtained by multiple rounds of strain mutagenesis of the present disclosure is used to ferment and reduce δ-lactone of 5-hydroxy-2-decenoic acid and δ-lactone of 5-hydroxy-2-dodecenoic acid in Massoia Oil. Which has a higher effectiveness, thus the production cost is greatly reduced. It has good industrial application prospects.

The aforementioned embodiments are merely some embodiments of the present disclosure, and the scope of the disclosure is not limited thereto. Thus, it is intended that the present disclosure cover any modifications and variations of the presently presented embodiments provided they are made without departing from the appended claims and the specification of the present disclosure.

## Claims

1. A method for preparing natural delta-decalactone and delta-dodecalactone by bioreduction of Massoia Oil, **characterized in that**, which comprises the following steps:
(1) culturing *Saccharomyces pastorianus* OMK-70 on a solid slant culture medium and a seed culture medium in sequence to obtain a seed culture solution, the *Saccharomyces pastorianus* OMK-70 was deposited in China Center for Type Culture Collection on December 27, 2019, with a deposit number of CCTCC NO: M 20191123;
(2) inoculating the seed culture solution into a fermentation medium and cultivating, adding Massoia oil in batches after strains grow to an early logarithmic stage, and continuously adding glucose and continually fermenting;
(3) adding butyl acetate into a culture solution obtained in step (2), completely stirring and reacting, leaving to stand to generate layers, and then acquiring an upper oil phase; and
(4) recycling the butyl acetate in the upper oil phase, rectifying a pot bottom by controlling a rectification temperature, and collecting fractions at different temperatures to obtain the natural delta-decalactone and the delta-dodecalactone.

2. The method according to claim 1, **characterized in that**, which comprises the following steps
(1) strain activation: streaking the *Saccharomyces pastorianus* OMK-70 from a glycerol tube preserved at a lower temperature on the solid slant culture medium and static culturing at 28 °C-30 °C for 48-72 hours to obtain the activated strains;
(2) preparation of the seed culture solution: inoculating the activated strains obtained in step (1) into the seed culture medium and shake culturing at 28 °C-30 °C and 200-250 rpm to grow to the early logarithmic stage to obtain the seed culture solution,
(3) fermentation and conversion: inoculating the seed culture solution obtained in step (2) into the fermentation medium at a volume ratio of 5-10% and cultivating for 6-12 hours in a condition that pH is 4.8-6.4, 28 °C-30 °C, a stirring speed is 100-600 rpm, and an aeration volume is 1:0.5, adding the Massoia oil in batches after the strains grow to the early logarithmic stage, and continuously adding glucose and continually fermenting for 48-72 hours;
(4) extraction: adding the butyl acetate to a culture broth obtained in step (3) at a volume ratio of 0.8-1.2:0.8-1.2, stirring at 30 °C-40 °C and 200-300 rpm for 1-2 hours, leaving to stand to generate the layers, and then acquiring the upper oil phase;
(5) solvent recycle: transferring the upper oil phase obtained in step (4) to a distillation flask to recycle the butyl acetate at an atmosphere pressure to obtain the pot bottom; and
(6) rectification: transferring the pot bottom obtained in step (5) into a rectification tower and rectifying by controlling the rectification temperature, and collecting the fractions at the different temperatures to obtain the natural delta-decalactone and the delta-dodecalactone.

3. The method according to claims 1 and/or 2, **characterized in that**: a composition of the solid slant culture medium in mass percentage comprises: peptone 0.5-5%, yeast extract 0.1-3%, glucose 1-5%, agar 0.5-5%, and a solvent is water.

4. The method according to claim 3, wherein the composition of the solid slant culture medium in mass percentage comprises: peptone 2.5%, yeast extract 1.5%, glucose 2.5%, agar 1.5%, and a balance is the water.

5. The method according to any one or more of claims 1 to 4, **characterized in that**: a composition of the seed culture medium in mass percentage comprises: peptone 0.5-5%, yeast extract 0.1-3%, and glucose1-5%, and a solvent is water.

6. The method according to claim 5, wherein the composition of the seed culture medium in mass percentage comprises: peptone 2.5%, yeast extract 1.5%, glucose 2.5%, and a balance is the water.

7. The method according to any one or more of claims 1 to 6, **characterized in that**: a composition of the fermentation medium in mass percentage comprises: glucose 2.0-5.0%, ammonium dihydrogen phosphate 0.5-1.0%, potassium dihydrogen phosphate 0.1-0.5%, magnesium sulfate 0.05-0.2%, calcium sulfate 0.05-0.1%, yeast extract powder 0.1-1.0%, and a solvent is water.

8. The method according to claim 7, wherein the composition of the fermentation medium in mass percentage comprises: glucose 2%, ammonium dihydrogen phosphate 1%, potassium dihydrogen phosphate 0.5%, magnesium sulfate 0.2%, calcium sulfate 0.1%, yeast extract powder 0.5%, and a balance is the water.

9. *Saccharomyces pastorianus* OMK-70, **characterized in that**: which deposited in China Center for Type Culture Collection on December 27, 2019, with a deposit number of CCTCC NO: M 20191123.

10. A method for preparing natural delta-decalactone and delta-dodecalactone by bioreduction of Massoia oil using the *Saccharomyces pastorianus* OMK-70 according to claim 9.
